# EUROPEAN PATENT APPLICATION

(11) **EP 1 634 870 A1**
(43) Date of publication of application: **15.03.2006**
(21) Application number: 04020350.7
(22) Date of filing: 27.08.2004
(51) Int. Cl.: C07D 209/24, C07F 9/40, A61K 31/405

(54) **Process and intermediates for the selective synthesis of Fluvastatin**

(71) Applicant: Zhejiang Hisun Pharmaceutical Co. Ltd., Jiaojiang District Taizhou City Zhejiang Province (CN); Tiefenbacher Pharmachemikalien Alfred E.Tiefenbacher GmbH & Co.KG, 22767 Hamburg (DE)
(72) Inventor: Guorong, Zhu, Zhejiang Province 318000 (CN); Hongquan, Gong, Zhejiang Province 318000 (CN); Becker, Stefan, Dr., 20251 Hamburg (DE)
(74) Representative: Hamm, Volker

(57) **Abstract**

The invention relates to process for the selective preparation of 3-hydroxy-6-dialkoxyphosphoryl-5-oxo-hexanoic acid esters, comprising a first step, in which a methylphosphonic acid dialkylester is treated with a base, and a second step, in which the product of the primary reaction is reacted with an optionally 3-protected 3-hydroxy-1,5-pentanoic diacid ester.

## Description

The present invention is directed to a new procedure for the selective synthesis of *syn-(E)-*7-[3-(4-fluorophenyl)-1-isopropyl-2-indolyl]-3,5-dihydroxy-6-heptenoic acid (Fluvastatin, **I**) as well as its pharmaceutically acceptable esters and salts. In particular, the present invention pertains to a procedure for the selective preparation of the sodium salt of Fluvastatin **(I-Na),** which is utilised to lower elevated general cholesterol-levels as well as elevated LDL-cholesterol-levels in the blood serum.

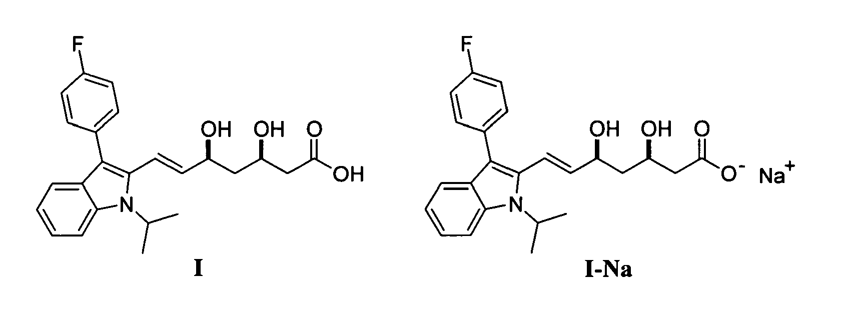

A particular problem in the preparation of compound **I** is the formation of the side chain's 3,5-dihydroxy-group, which, necessarily, has to be present as the *syn*-stereoisomer, thus, the 3*R*,5*S*/3*S*,5*R*-pair of enantiomers. The undesired *anti*-enantiomers may only be present in a very low concentration; according to a draft of the US-Pharmacopoeia (Pharmacopeial Previews, Pharmacopeial Forum, Vol. 25, No. 4, 8420-8426), the pharmaceutical compound Fluvastatin-Na should not comprise more than 0,8% of its *anti-*isomer*.*

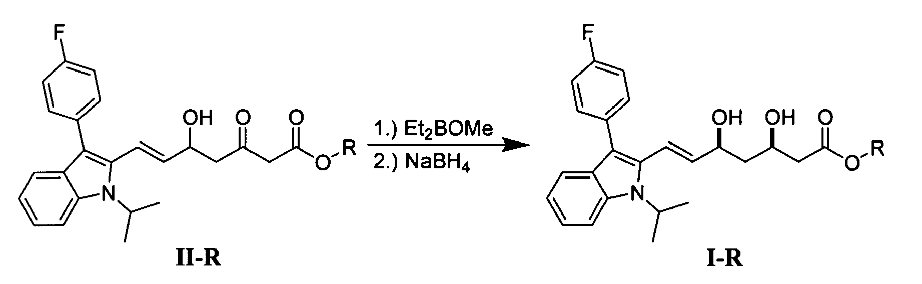

From the state of the art, methods for the preparation of Fluvastatin and its sodium salt are already known. According to the scientific publication K.-M. Chen et al., *Tetrahedron Letters* **1987,** Vol. 28, No. 2, 155-188, Fluvastatin methyl ester can be obtained by mixing *(E)-*7-[3-(4-fluorophenyl)-1-isopropyl-2-indolyl]-5-hydroxy-3-oxo-6-heptenoic acid methyl ester **(II-**Me) and methoxy diethylborane (Et₂BOMe) in a fist step, and subsequently reducing the resulting complex with sodium borohydride (NaBH₄). Utilisation of this method results in an overall yield of 90% and a *syn*/*anti*-ratio of 98:2 is obtained after a substantial reaction time of 5 hours. Obviously, the low selectivity of this process makes further means for the purification of the primary reaction product necessary.

European patent application EP-A-0 363 934 describes a similar, but improved protocol, wherein a 5-hydroxy-3-oxo-6-heptenoic acid ester derivative **II-R,** is reduced by addition of a previously prepared mixture of methoxy diethylborane and sodium borohydride in THF/methanol. According to this document's teaching, changing the order, in which the borane compounds are added (compared to the procedure of K.-M. Chen et al., *Tetrahedron Letters* **1987*,*** Vol. 28, No. 2, 155-158) highly improves the reduction's selectivity. Unfortunately, this procedure also yields acceptable selectivities only in those cases, which utilise the *tert*-butylester **(II-*tert*-Bu).** Application of these *tert*-butylesters, though, is generally accompanied by several severe problems, such as the problematic removal of *tert*-butanol after saponification of the esters in the synthesis of Fluvastatin-Na. Utilising the methyl esters (**II-Me**), which are highly advantageous for industrial processes, an overall yield of only 80% and a selectivity of only 99,07% are obtained. Therefore, before the final products of the reaction sequence described in EP-A-0 363 934 could be used as active ingredients in pharmaceutical compositions, the stereoisomers have to be separated ( the *anti*-isomers have to be removed).

Due to the large amounts handled in macroscale industrial processes, such a separation of stereoisomers is extremely arduous, if possible at all. Re-crystallisations and chromatographic separations increase e.g., the consumption of solveins which is not only economically, but also ecologically highly disadvantageous.

It is an object of the present invention to provide a process for the selective synthesis of *syn-*(E)-7-[3-(4-fluorophenyl)-1-isopropyl-2-indolyl]-3,5-dihydroxy-6-heptenoic acid (Fluvastatin, **I)** as well as its esters **(I-R)** and salts, especially its sodium salt **(I-Na),** which yields the syn-compounds with high selectivity, such that no further separation of the stereoisomers is necessary before the final products are used in pharmaceutical compositions. It is a further object of the present invention to provide the starting materials for the highly *syn*-selective process according to the invention.

These objects are attained by the features of the independent claims. The dependent claims define preferred embodiments of the present invention.

By the processes disclosed in the present invention for the first time *syn*-selectivities are obtained, which allow the products to be directly used as active ingredients in pharmaceutical compositions without further removal of the undesired diastereomeric material. By the process according to the present invention *syn*/*anti*-selectivities of at least 99,2/0,8 are obtained; preferably 99,5:0,5. According to an especially preferred embodiment, the ratio of the *syn-*enantiomers is 99,8%.

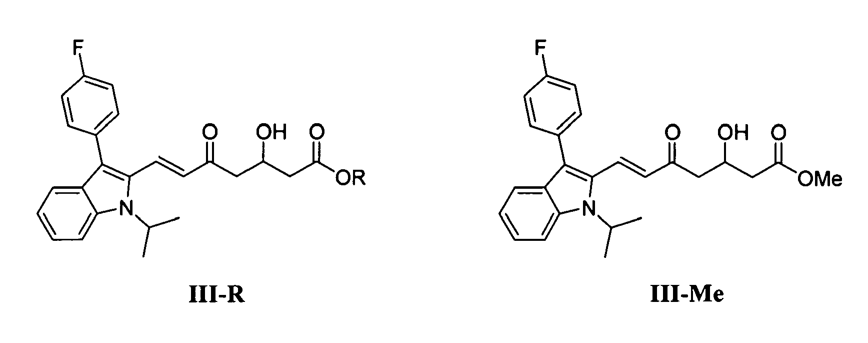

The synthetic strategies utilised in industrial processes known from the state of the art, the selectivities of which do not allow direct utilisation of the product as pharmaceutical compounds, all start from derivatives of *(E)*-7 -[3-(4-fluorophenyl)-1-isopropyl-2-indolyl]-5-hydroxy-3-oxo-6-heptenoic acid **(II)** or rather its esters **(II-R).** Now, it has been surprisingly found that by using *(E)*-7-[3-(4-fluorophenyl)-1-isopropyl-2-indolyl]-3-hydroxy-5-oxo-6-heptenoic acid esters **(III-R)** the selectivity is highly improved, so that the product can be used directly without any separation of the *syn*/*anti*-stereo isomers. Even the use of methyl esters, which generally shows great advantages in industrial processes but so far did not yield sufficient selectivities, leads to acceptable selectivities. The process according to the present invention gives high yields known from the state of the art after very short reaction times.

The starting material for the highly selective reductions of the present invention, the 3-hydroxy-5-oxo-6-heptenoic acid esters **(III-R)** can be obtained by Wittig or Wittig analog reactions, thus, by coupling a carbaldehyde to a hexanoic acid ester having a functional group in 6-position, which can be activated to an ylide or a carbanion. Suitable 3-protected 3-hydroxy-5-oxo-hexanoic acid esters having a 6-phosphor-function can be obtained by chain elongation of an asymmetric 3-protected 1,5-pentanoic diacid monoalkylester, e.g., with a deprotonated methylphosphonic acid ester. According to the present invention, other groups, leading to 6-phosphor-ylides or 6-phosphor-carbanions of 3-hydroxy-5-oxo-hexanoic acids, are equally suitable. As protection groups for the 3-hydroxy functional group every suitable protection group can be utilised. According to the present invention, silylethers, such as TMS-(trimethylsilyl-), TBDPS-(*tert*-butyldiphenylsilyl-), or TBS-(*tert*-butyldimethylsilyl-) ethers, are preferred as protection groups for the 3-hydroxy group; with the TBS-protection group being especially preferred.

Asymmetric 3-protected 1,5-pentanoic diacid monoalkylesters, such as the monomethylester, are obtainable by hydrolysis of the 3-protected 1,5-pentanoic diacid anhydrates with the corresponding alkylalcoholates, such as methanol.

The functional groups in 6-position of the hexanoic acid derivatives are preferably phosphonium salts, which can be activated to the corresponding ylides, and which can be coupled to the aldehyde via Wittig-reaction. In another preferred embodiment of the present invention, the functional group in 6-position of the hexanoic acid derivative is a phosphonic acid ester, which can be deprotonated to the phosphonate-carbanion, and which can be coupled to the aldehyde by a Homer-Wadsworth-Emmons-reaction; in an especially preferred embodiment the 6-functional group is a phosphonic acid dialkylester, such as the dimethoxy phosphoryl-group.

After chain elongation, the free acid function can be protected, preferably as an ester. The respective protection is preferably carried out by reaction with an alkylalkalimetal compound in the presence of a base; especially preferred by a reaction with methyllithium in the presence of anhydrous potassium carbonate.

For the activation of the 6-phosphoric function of the 3-protected-3-hydroxy-5-oxohexanoic acid derivatives, every suitable base, such as alkylalkalimetal compounds, alkalimetal hydrides, or alkalimetal alcoholates, can be used. In a preferred embodiment alkylalkalimetal compounds are used; in an especially preferred embodiment *n*-butyllithium.

Coupling of the 3-hydroxy-5-oxo-hexanoic acid derivatives to the corresponding [3-(4-fluorophenyl)-1-isopropyl-2-indolyl]-carbaldehyde and deprotection of the 3-hydroxy function are carried out as known from the art. In the case silyl-protection groups are used, deprotections by addition of fluoride compounds are preferred. In an especially preferred embodiment of the present invention the deprotection is carried out by addition of ammonium hydrofluoride.

The stereoselective reduction of the (*E*)-7-[3-(4-fluorophenyl)-1-isopropyl-2-indolyl]-3-hydroxy-5-oxo-6-heptenoic alkylesters **(III-R)** obtained by the above process is carried out by mixing the species to be reduced **III-R** with an alkoxydialkylborane, whereby, without being committed to that, a borane/boronate-complex is formed. The alkoxydialkylborane can be used ready-made or it may be prepared in situ from a trialkylborane; preferably it is generated from a trialkylborane by introduction of air in the presence of an alcohol. In a preferred embodiment, methoxydialkylborane is prepared by introduction of air into a solution of a trialkylborane in a solvent mixture comprising methanol. In another preferred embodiment methoxydiethylborane is prepared in situ by introduction of air into a solution of a triethylborane in a methanol/THF mixture before the 3-hydroxy-5-oxo-compound is added.

In the next step, this boronate-complex is reduced by addition of a reducing agent. Especially suitable solvents in these reactions are alcohols possibly in admixture with ethers, preferably with tetrahydrofurane (THF). According to the present invention, reductions carried out in mixtures of THF with a C₁-C₄-alcohol (linear or branched) are preferred; especially preferred are mixtures of methanol with THF. The alkoxydialkylborane can be added directly or can be formed in situ. As reducing agents, every suitable reducing agent can be used; according to the present invention utilisation of sodium borohydride (NaBH₄) is preferred.

The (*E*)-7-[3-(4-fluorophenyl)-1-isopropyl-2-indolyl]-3,5-dihydroxy-6-heptenoic acid esters **(I-R),** which are obtainable highly *syn*-selective by the reaction sequence described above, can be transformed into the corresponding free acid (Fluvastatin, I). Additionally, they can be transformed directly into their pharmaceutically acceptable salts by addition of a base, especially into the sodium salt of Fluvastatin **(I-Na)** by saponification with sodium hydroxide.

The present invention is further explained by the following examples without being limited to them.

### Example 1: 3-tert-butyldimethylsilanyloxy-1,5-pentanoic diacid monomethylester (S2)

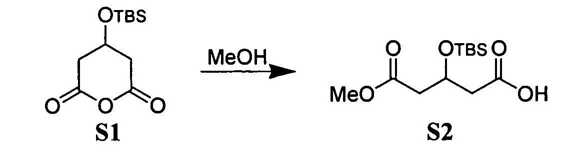

Dissolve 18,0 kg 3-hydroxy-TBS-ether-1,5-pentanoic diacid anhydrate **(S1)** in 90 1 methanol under the protection of nitrogen gas and reflux for 24 h. The reaction is observed by TLC. When the starting material has disappeared (as detected by TLC), the reaction mixture is concentrated to dryness and 20,0 kg of the title compound are obtained.

### Example 2: 3-tert-butyldimethylsilanyloxy-6-methoxyphoshoryl-5-oxo-hexanoic acid (S3)

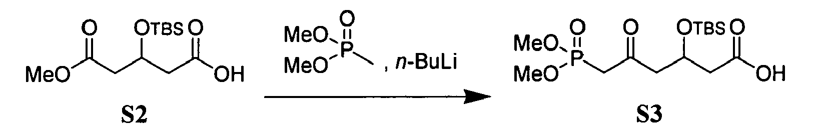

Dropwise add 168,6 1 *n*-butyllithium/diethylether solution (1,72 N) to a mixture of THF and 35,9 kg methyl phosphonic acid dimethylester within 15 minutes at ―78 C°, react while stirring for 30 minutes, then dropwise add a mixture of 20,0 kg 3-*tert*-butyldimethylsilanyloxy-1,5-pentanoic diacid monomethylester (**S2**) in THF within 5 minutes. React while stirring for 3 hours at ―78 C°, and dropwise add saturated ammonium hydrochloride solution. Subsequently, acidify the reaction mixture with 2 N hydrochloric acid and extract with ethyl acetate. Combine the organic layers and wash with brine, dry with anhydrous sodium sulphate, and remove the solvents under vacuum. 27,6 kg of the raw title compound S3 are obtained.

### Example 3: 3-tert-butyldimethylsilanyloxy-6-dimethoxyphosphoryl-5-oxo-hexanoic methylester (S4)

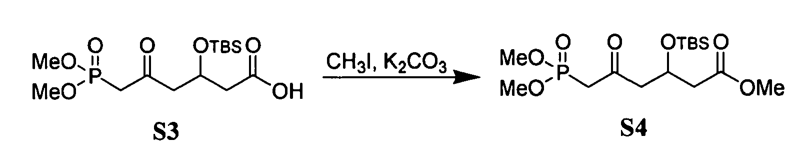

27,6 kg 3-*tert*-butyldimethylsilanyloxy-6-dimethoxyphosphoryl-5-oxo-hexanoic acid **(S3)** are dissolved in 220 1 acetone. 53,3 kg iodomethane and 10,35 kg anhydrous potassium carbonate are added. The reaction mixture is stirred for 24 hours at room temperature and observed by TLC. After the reaction has finished water is added. The reaction mixture is extracted with ethyl acetate and the combined organic layers are washed with brine and dried with anhydrous sodium sulphate. After removal of the solvent under vacuum, 24,7 kg of the title compound **S4** are obtained.

### Example 4: (E)-7-[3-(4-fluorophenyl)-1 -isopropyl-2-indolyl]-3-tert-butyldimethylsilanyloxy-5-oxo-6-heptenoic acid methylester (F2)

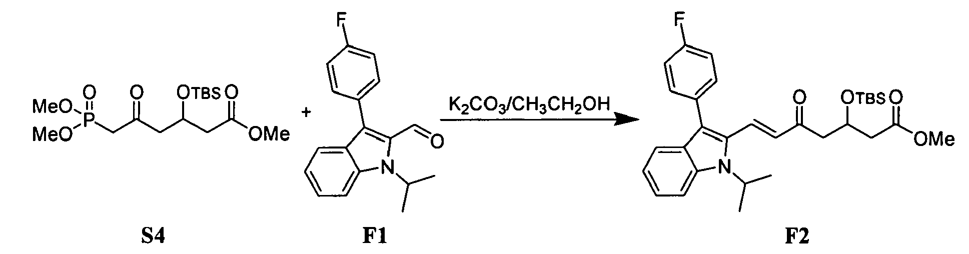

Under the protection of nitrogen gas, 8,9 kg potassium carbonate and 15,1 kg [3-(4-fluorophenyl)-1-isopropyl-2-indolyl]-carbaldehyde **(F1)** are added to a solution of 24,7 kg 3-*tert*-butyldimethylsilanyloxy-6-dimethoxyphosphoryl-5-oxo-hexanoic acid methylester in 150 1 anhydrous ethanol. The reaction mixture is agitated for 24 hours, observed by HPLC, and extracted with ethyl acetate and brine. The layers are separated and the aqueous phase is extracted with ethyl acetate. The combined organic phases are washed with brine, dried with anhydrous sodium sulphate, and the solvent is removed under vacuum. After recrystallisation from hexane/ethyl acetate (5:1) 18,2 kg of the title compound **F2** (63,1%) are obtained.

### Example 5: (E)-7-[3-(4-flurophenyl)-1-isopropyl-2-indolyl]-3-hydroxy-5-oxo-6-heptenoic acid methyl ester (III-Me)

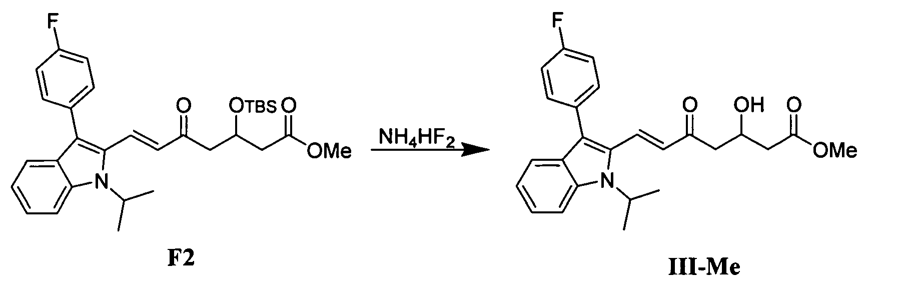

18,2 kg of the TBS-protected compound **F2** are dissolved in acetic acid and 3,9 kg ammonium hydrofluoride are added under protection of nitrogen gas. The reaction mixture is agitated for 24 hours, observed by HPLC, and extracted with water and ethyl acetate. The combined organic layers are washed with water and sodium bicarbonate solution until the pH-value is greater than 7. The organic solution is dried with anhydrous sodium sulphate and concentrated under vacuum. After crystallisation from *n*-hexane 12,2 kg of the title compound **III-Me** (85,1 %) are obtained.

### Example 6: (E)-7-[-3-(4-fluorophenyl)-1-isopropyl-2-indolyl]-3,5-dihydroxy-6-heptenoic methyl ester (I-Me)

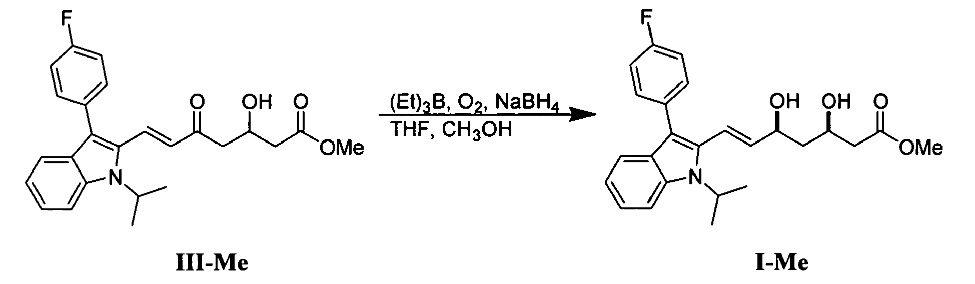

45,2 kg of a triethylborane solution in THF are added to a mixture of 1201 THF and 441 anhydrous methanol. Air is introduced for 2 minutes and the reaction mixture is agitated for 30 minutes. 12,2 kg of the 3-hydroxy-5-oxo-compound **III-Me** are added and the mixture is stirred for 30 minutes until the solids are dissolved completely. The reaction mixture is cooled to ―78 - ―80C° and 1,524 kg sodium borohydride are added. After completion of the reaction, ethyl acetate and sodium bicarbonate solution are added slowly. The organic layer is separated and the aqueous phase is extracted with ethyl acetate. The combined organic layers are washed with brine, dried with sodium sulphate, and the solvent is removed under vacuum. The concentrate is dissolved in methanol, repeatedly, and evaporated to dryness. The obtained solid is dissolved in anhydrous diethyl ether under reflux and crystallised to obtain 10,4 kg of the title compound I-Me (84,8%).

### Example 7: Fluvastatin-Na (I-Na)

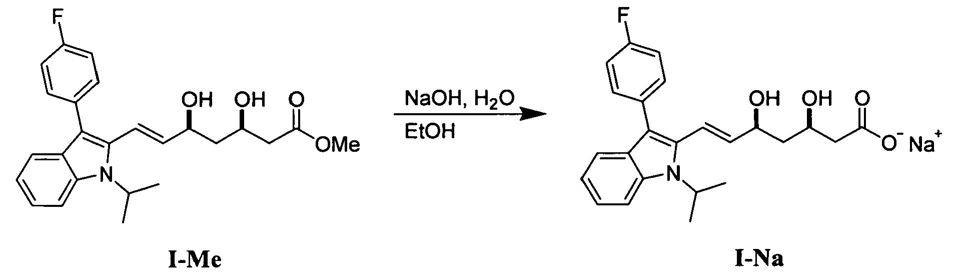

10.4 kg of the Fluvastatin methylester **I-Me** is dissolved in 50 l ethanol and 24,6 1 1 N so-diumhydroxide solution. The reaction mixture is agitated for 30 minutes and observed by HPLC. After completion of the reaction the solvent is removed under vacuum. The obtained solid si dissolved in water and extracted with diethyl ether. After lyophilisation of the aqueous phase 10,0 kg Fluvastatin-Na **(I-Na)** are obtained.

## Claims

1. Process for the selective preparation of 3-hydroxy-6-dialkoxyphospho-ryl-5-oxo-hexanoic acid esters, which are optionally protected in 3-position, comprising a first step, in which a methylphosphonic acid dialkylester is treated with a base, and a second step, in which the product of the primary reaction is reacted with an optionally 3-protected 3-hydroxy-1,5-pentanoic diacid ester.

2. Process according to claim 1, wherein the 3-protection group is a silyl-protection group.

3. Process according to claim 1 or 2, wherein the base is an alkyllithium compound.

4. Process according to claims 1-3, wherein the methylphosphonic acid dialkylester is methylphosphonic acid dimethylester.

5. Process for the preparation of (*E*)-7-[3-(4-fluorophenyl)-1-isopropyl-2-indolyl]-3-hydroxy-5-oxo-6-heptenoic acid esters, comprising a reaction of a 3-hydroxy-6-dialkoxyphosphoryl-5-oxo-hexanoic acid ester, optionally protected in 3-position and obtained by one of the process of claims 1-4, with [3-(4-fluorophenyl)-1-isopropyl-2-indolyl]-carbaldehyde.

6. Process for the preparation of (*E*)-7-[3-(4-fluorophenyl)-1-isopropyl-2-indolyl]-3,5-dihydroxy-6-heptenoic acids as well as their pharmaceutically acceptable esters and salts comprising in a first step a reaction of an (*E*)-7-[3-(4-fluorophenyl)-1-isopropyl-2-indolyl]-3-hydroxy-5-oxo-6-heptenoic acid ester, obtained according to claim 5, with an alkoxydialkylborane and subsequent treatment with a reducing agent.

7. Process for the preparation of (*E*)-7-[3-(4-fluorophenyl)-1-isopropyl-2-indolyl]-3,5-dihydroxy-6-heptenoic acids as well as their pharmaceutically acceptable esters and salts according to claim 6, wherein the alkoxydialkylborane is generated in situ from a trialkylborane.

8. Process for the preparation of *(E)*-7-[3-(4-fluorophenyl)-1-isopropyl-2-indolyl]-3,5-dihydroxy-6-heptenoic acids as well as their pharmaceutically acceptable esters and salts, **characterised by** the reduction of a *(E)*-7-[3-(4-fluorophenyl)-1-isopropyl-2-indolyl]-3-hydroxy-5-oxo-6-heptenoic acid ester in the presence of a alkoxydialkylborane, wherein, if necessary, the acid is set free or the reduction product is saponificated to its salt.

9. Process according to one of claims 6 to 8, wherein the primary reduce-tion product is saponificated to its sodium salt.

10. Process according to one of claims 6 to 9, wherein the *(E)*-7-[3-(4-fluorophenyl)-1-isopropyl-2-indolyl]-3-hydroxy-5-oxo-6-heptenoic acid ester is the methylester.

11. Process according to one of claims 6 to 10, wherein the alkoxydialkylborane and the (*E*)-7-[3-(4-fluorophenyl)-1-isopropyl-2-indolyl]-3-hydroxy-5-oxo-6-heptenoic acid esters are added in a first step and the reduction agent is added, subsequently.

12. Process according to one of claims 6 to 11, wherein the alkoxydialkylborane is methoxydiethylborane.

13. Process according to one of claims 6 to 12, wherein the reduction agent is sodiumborohydride.

14. Use of 3-hydroxy-6-dialkoxyphosphoryl-5-oxo-hexanoic esters, optionally protected in 3-position in the preparation of Fluvastatin as well as its pharmaceutically acceptable esters and salts.

15. Use of (*E*)-7-[3-(4-fluorophenyl)-1-isopropyl-2-indolyl]-3-hydroxy-5-oxo-6-heptenoic acid esters for the preparation of Fluvastatin as well as its pharmaceutically acceptable esters and salts.

16. Sodium salt of Fluvastatin obtained by one of the claims 1-13.

17. Use of the Fluvastatin sodium salt according to claim 16 for the preparation of pharmaceutical compositions for the treatment of elevated cholesterol-levels in the blood serum.

18. *(E)*-7-[3-(4-flurophenyl)-1-isopropyl-2-indolyl]-3-hydroxy-5-oxo-6-heptenoic acid methyl ester **(III-Me).**

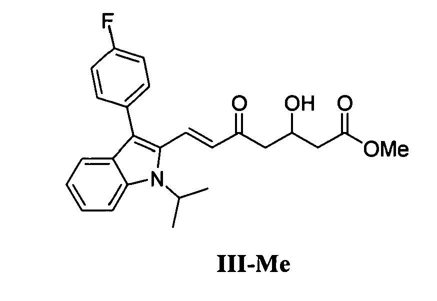

19. Use of *(E)*-7-[3-(4-flurophenyl)-1-isopropyl-2-indolyl]-3-hydroxy-5-oxo-6-heptenoic acid methyl ester **(III-Me)** in the synthesis of Fluvastatin as well as its pharmaceutically acceptable esters and salts.

20. (*E*)-7-[3-(4-fluorophenyl)-1-isopropyl-2-indolyl]-3-*tert*-butyldimethyl-silanyloxy-5-oxo-6-heptenoic acid methylester **(F2).**

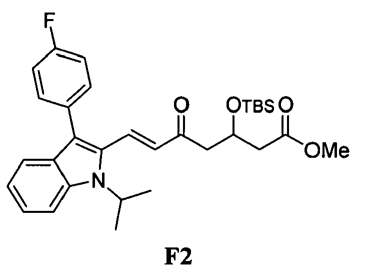

21. Use of *(E)*-7-[3-(4-fluorophenyl)-1-isopropyl-2-indolyl]-3-*tert*-butyldi-methylsilanyloxy-5-oxo-6-heptenoic acid methylester **(F2)** in the synthesis of Fluvastatin as well as its pharmaceutically acceptable esters and salts.
